# FASCICULE DE BREVET EUROPEEN

(11) **EP 1 243 251 B1**
(45) Date de publication et mention de la délivrance du brevet: **24.05.2006**
(21) Numéro de dépôt: 02290536.8
(22) Date de dépôt: 05.03.2002
(51) Int. Cl.: A61K 8/06, A61K 8/81, A61K 8/88, A61Q 1/00, A61Q 5/00, A61Q 19/00

(54) **Composition contenant des fibres, et ses utilisations notamment cosmétiques**
Zusammensetzung,die Fasern enthält und deren Verwendungen insbesondere in der Kosmetik
Composition containing fibres and its uses particularly in cosmetics

(30) Priorité: 20.03.2001 FR 0103767
(43) Date de publication de la demande: 25.09.2002
(73) Titulaire: L'OREAL, 75008 Paris (FR)
(72) Inventeur: Chevalier, V-ronique, 94440 Villecresnes (FR); Agostini, Albane, Appartement 38C, 50100 Cherbourg-Octeville (FR)
(74) Mandataire: Rasson, Catherine

(56) Documents cités:
- EP-A- 0 106 762
- EP-A- 1 053 742

## Description

La présente demande se rapporte à une composition comprenant dans un milieu physiologiquement acceptable, une phase huileuse dispersée dans une phase aqueuse, des fibres, des particules sphériques et des plaquettes, et à l'utilisation de la dite composition, en particulier pour le soin, le traitement et/ou le maquillage de la peau du corps ou du visage, des cheveux, des cils et/ou des lèvres.

Il est connu par le document JP07-196440, des compositions cosmétiques contenant des fibres de polyamide courtes, celles-ci donnant aux dites compositions un toucher velouté et une bonne tenue cosmétique. Toutefois, l'incorporation de fibres, et en particulier de ces fibres de polyamide, dans les dispersions comprenant une phase huileuse dispersée dans une phase aqueuse, et en particulier dans les émulsions huile dans eau (H/E) qui sont des dispersions stabilisées par des émulsionnants, pose des problèmes lors de l'application de la composition sur les matières kératiniques telles que la peau, et ce notamment quand la quantité de fibres est importante. En effet, les fibres, notamment quand elles sont en quantité assez importante, ont tendance à s'agréger dans les émulsions H/E les contenant. Par ailleurs, ces émulsions contenant des fibres ont tendance à « boulocher » sur la peau, c'est-à-dire à s'appliquer par paquets en faisant comme des petites boules sur la peau.

Le document EP-A-0 106 762 révèle l'association de fibres de polyéthylène avec de la silice, de préférence amorphe et synthétique, dans un produit cosmétique de maquillage.

Il subsiste donc le besoin de dispersions H/E et notamment d'émulsions H/E contenant des fibres, et notamment des fibres de polyamide, même en quantité importante, et n'ayant pas les inconvénients de l'art antérieur.

La demanderesse a découvert de façon inattendue que le fait d'associer aux fibres, des particules sphériques et des plaquettes permettaient d'éviter les problèmes de boulochage et d'agrégation, dus aux fibres, et de réaliser des émulsions huile-dans-eau contenant des fibres, ne posant pas de problème lors de l'application sur la peau, même en présence d'une quantité importante de fibres.

La présente invention concerne une composition comprenant dans un milieu physiologiquement acceptable, une phase huileuse dispersée dans une phase aqueuse, des fibres, des particules sphériques et des plaquettes.

On entend ici par « milieu physiologiquement acceptable », un milieu compatible avec les matières kératiniques et notamment la peau, les lèvres, le cuir chevelu, les cils, les yeux et/ou les cheveux.

La dispersion peut être plus particulièrement une émulsion H/E.

L'association utilisée permet non seulement d'éviter que les fibres ne s'agglomèrent, mais encore de faciliter l'application de la composition les contenant, sur les matières kératiniques et en particulier sur la peau. Ainsi, la composition selon l'invention ne « bouloche » pas et donc, ne fait pas des petites boules lors de l'application sur la peau. En outre, elle se délite facilement, c'est-à-dire qu'elle s'applique et se dépose en une quantité suffisante et de façon homogène sur la peau ou la matière kératinique où elle est appliquée. Cet effet de l'association des plaquettes et de particules sphériques se vérifie pour toute composition contenant des fibres et notamment pour les dispersions de l'invention.

L'invention a encore pour objet l'utilisation cosmétique de l'association de plaquettes et de particules sphériques dans une composition cosmétique contenant des fibres, pour éviter que les fibres ne s'agglomèrent, pour éviter le boulochage de la composition et pour faciliter l'application (et le délitage) de la composition sur les matières kératiniques et notamment la peau.

On entend dans la présente demande par « particules sphériques » des particules ayant la forme ou sensiblement la forme d'une sphère, insolubles dans le milieu de la composition selon l'invention, même à la température de fusion du milieu (environ 100°C).

On entend en outre ici par « plaquettes » ou « lamelles » des particules de forme parallélépipédique (surface rectangulaire ou carrée), discoïdale (surface circulaire) ou ellipsoïdale (surface ovale), caractérisées par trois dimensions : une longueur, une largeur et une hauteur, particules qui sont insolubles dans le milieu de la composition selon l'invention, même à la température de fusion du milieu (environ 100°C).

La composition de l'invention peut comprendre une ou plusieurs sortes de fibres, une ou plusieurs sortes de particules sphériques et une ou plusieurs sortes de plaquettes.

### Fibres

Les fibres utilisables dans la composition de l'invention peuvent être des fibres hydrophiles ou hydrophobes, d'origine synthétique ou naturelle, minérale ou organique.

Ces fibres peuvent être courtes ou longues, unitaires ou organisées par exemple tressées. Elles sont de forme généralement cylindrique contrairement aux plaquettes de forme parallélépipédique et aux particules sphériques de forme sphérique. Leur morphologie peut être quelconque et notamment de section circulaire ou polygonale (carrée, triangulaire, hexagonale ou octogonale) selon l'application spécifique envisagée. En particulier, leurs extrémités sont épointées et/ou polies pour éviter de se blesser.

En particulier, les fibres peuvent avoir une longueur (L) allant de 1 µm (0,001 mm) à 10 mm, de préférence de 0,1 µm à 5 mm et mieux de 0,1 mm à 1,5 mm. Leur section peut être comprise dans un cercle de diamètre (D) allant de 1 nm (0,001 µm) à 100 µm, de préférence allant de 1 nm (0,001 µm) à 50 µm et mieux de 5 µm à 40 µm.

De préférence, les fibres utilisées selon la présente invention ont un facteur de forme, c'est-à-dire un rapport L/D (longueur/diamètre) allant de 3,5 à 2500, mieux de 5 à 500 et encore mieux de 5 à 150.

Le titre des fibres est souvent donné en denier ou décitex. Le denier est le poids en gramme pour 9 km de fil. De préférence, les fibres utilisées selon l'invention ont un titre allant de 0,15 à 30 deniers, et mieux de 0,18 à 18 deniers.

Le facteur de forme, le titre et la morphologie des fibres sont les trois facteurs importants pour définir une fibre.

Les fibres peuvent être celles utilisées dans la fabrication des textiles et notamment des fibres de soie, de coton, de laine, de lin, de cellulose extraites notamment du bois, des légumes ou des algues, de polyamide (Nylon® ), de cellulose modifiée (rayonne, viscose, acétate notamment d'acétate de rayonne), de poly-p-phénylène téréphtalamide notamment de Kevlar® , en acrylique notamment de polyméthacrylate de méthyle ou de poly 2-hydroxyéthyl méthacrylate, de polyoléfine et notamment de polyéthylène ou de polypropylène, de verre, de silice, d'aramide, de carbone notamment sous forme graphite, de Téflon® , de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-dessus, comme des fibres de polyamide/polyester.

Comme fibres de polyuréthane, on peut citer par exemple les fibres en polymère poly(uréthane-urée), appartenant à la classe des élastanes, et notamment celles commercialisées sous la dénomination LYCRA® par la société DuPont.

On peut aussi utiliser les fibres synthétiques résorbables utilisées en chirurgie, comme les fibres préparées à partir d'acide glycolique et de caprolactone (Monocryl de la société Johnson & Johnson) ; les fibres synthétiques résorbables du type copolymère d'acide lactique et d'acide glycolique (Vicryl de la société Johnson & Johnson); les fibres de polyester téréphtalique (Ethibond de la société Johnson & Johnson) et les fils d'acier inoxydable (Acier de la société Johnson & Johnson).

On peut aussi utiliser des mélanges des fibres citées ci-dessus.

Par ailleurs, les fibres peuvent être traitées ou non en surface, enrobées ou non. Il peut s'agir notamment de fibres enrobées et/ou fonctionnalisées, « fonctionnalisées » signifiant que les fibres sont traitées en surface afin d'en modifier les propriétés.

Comme fibres enrobées utilisables dans l'invention, on peut citer des fibres de polyamide enrobées de sulfure de cuivre pour un effet antistatique (par exemple R-STAT de la société Rhodia) ou un autre polymère permettant une organisation particulière des fibres (traitement de surface spécifique) ou traitement de surface induisant des effets de couleurs/hologrammes (fibre Lurex de la société Sildorex, par exemple).

Les fibres peuvent être également fonctionnalisées, c'est-à-dire être modifiées de manière à avoir une fonction spécifique. Cette fonctionnalisation des fibres peut être réalisée aussi bien sur les fibres que dans les fibres, et ce par toute méthode permettant d'accrocher un composé aux fibres ou de le piéger dans les cavités formées par la géométrie des fibres. Comme méthodes, on peut citer par exemple l'enduction des fibres par un actif ; la fixation de particules renfermant un actif, telles que nanocapsules ou nanosphères, sur les fibres ; l'adsorption dans les fibres ; la fixation par réaction chimique. On peut ainsi utiliser des fibres ayant des fonctionnalités particulières, par exemple des fibres anti-UV par modification avec filtres solaires chimiques ou physiques ; des fibres bactéricides ou antiseptiques par modification avec des conservateurs ou d'anti-bactériens; des fibres colorées par modification avec des molécules colorantes; des fibres kératolytiques ou desquamantes par modification avec des agents kératolytiques ou desquamants; des fibres hydratantes par modification avec des agents hydratants ou des polymères rétenteurs d'eau ; les fibres parfumées par modification avec un parfum; des fibres analgésiques ou apaisantes par modification avec un anti-inflammatoire ou un agent apaisant ; des fibres anti-transpirantes par modification avec un anti-transpirant.

Selon leurs propriétés, les fibres utilisées selon la présente invention peuvent être introduites dans un milieu aqueux, un milieu huileux ou dans une poudre.

Les fibres utilisables selon l'invention sont de préférence choisies parmi les fibres de polyamide, les fibres de poly-p-phénylène téréphtalamide, les fibres de coton et leurs mélanges. Leur longueur peut aller de 0,1 à 10 mm, de préférence de 0,1 à 1 mm, leur diamètre moyen peut aller de 5 à 50 µm et le facteur de forme va de préférence de 5 à 150.

En particulier, on peut utiliser les fibres de polyamide commercialisées par les Etablissements P. Bonte sous le nom Polyamide 0,9 dtex 0,3 mm, ayant un diamètre moyen de 15 à 20 µm, un titre d'environ 0,9 dtex (0,81 denier) et une longueur allant de 0,3 mm à 1,5 mm. On peut aussi utiliser les fibres de poly-p-phénylène téréphtalamide de diamètre moyen de 12 µm et de longueur d'environ 1,5 mm comme celles vendues sous le nom de Kevlar Floc par la société Du Pont Fibres. Ces fibres de polyamide sont de préférence introduites dans un milieu huileux ou par voie sèche dans une poudre.

On peut aussi utiliser des fibres de coton ayant un diamètre moyen de 20 µm, une longueur de 0,3 mm, et un facteur de forme de 15, telles que celles commercialisées par la société Filature de Lomme, par l'Institut Textile de France, par la société Textiles des Dunes ou par la société Velifil.

Les fibres peuvent être présentes dans la composition selon l'invention en une quantité allant de 0,1 à 50 % en poids, de préférence de 0,5 à 30 % en poids, mieux de 1 à 20 % en poids et encore mieux de 2 à 15 % en poids par rapport au poids total de la composition.

### Particules sphériques

Les particules sphériques utilisées selon l'invention ont la forme ou sensiblement la forme d'une sphère et peuvent être creuses ou pleines. De façon avantageuse, les particules de l'invention ont une granulométrie (diamètre moyen en nombre) allant de 0,1 µm à 250 µm et encore mieux allant de 1 µm à 150 µm, et encore mieux de 10 µm à 100 µm.

Les particules sphériques peuvent être des microsphères organiques ou minérales. Comme particules sphériques utilisables dans la composition de l'invention, on peut citer par exemple la poudre de silice ; les particules de polyamide et notamment le NYLON 12 comme celui commercialisé sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto; les poudres de matériaux organiques naturels tels que les amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone ; et leurs mélanges.

Ces particules sphériques peuvent être présentes dans des quantités allant par exemple de 0,1 à 30 % en poids, de préférence de 0,5 à 25 % en poids et mieux de 1 à 10 % en poids par rapport au poids total de la composition.

### Plaquettes

Comme indiqué ci-dessus, les plaquettes sont des particules de forme parallélépipédique (surface rectangulaire ou carrée), discoïdale (surface circulaire) ou ellipsoïdale (surface ovale), caractérisées par trois dimensions : une longueur, une largeur et une hauteur. Quand la forme est circulaire, la longueur et la largeur sont identiques et correspondent au diamètre d'un disque, tandis que la hauteur correspond à l'épaisseur du disque. Quand la surface est ovale, la longueur et la largeur correspondent respectivement au grand axe et au petit axe d'une ellipse et la hauteur correspond à l'épaisseur du disque elliptique formé par la plaquette. Quand il s'agit d'un parallélépipède, la longueur et la largeur peuvent être de dimensions identiques ou différentes: quand elles sont de même dimension, la forme de la surface du parallélépipède est carrée ; dans le cas contraire, la forme est rectangulaire. Quant à la hauteur, elle correspond à l'épaisseur du parallélépipède.

La longueur des plaquettes utilisées selon l'invention va de préférence de 0,01 à 100 µm, mieux de 0,1 à 50 µm et encore mieux de 1 à 50 µm. La largeur de ces plaquettes va de préférence de 0,01 à 100 µm, mieux de 0,1 à 50 µm et encore mieux de 1 à 10 µm. Et la hauteur (épaisseur) de ces plaquettes va de préférence de 0,1 nm à 1 µm (0,1 à 1000 nm), mieux de 1 nm à 600 nm et encore mieux de 1 nm à 500 nm.

Comme plaquettes utilisables dans la composition de l'invention, on peut citer par exemple les pigments minéraux or organiques, les silicates lamellaires, et leurs mélanges.

Par pigments, il faut comprendre des particules blanches ou colorées, minérales ou organiques, insolubles dans le milieu de la composition, destinées à colorer et/ou opacifier la composition.

Comme pigments minéraux utilisables dans l'invention, on peut citer les oxydes de titane, de zirconium ou de cérium ainsi que les oxydes de zinc, de fer ou de chrome, le bleu ferrique, et leurs mélanges. Parmi les pigments organiques utilisables dans l'invention, on peut citer le noir de carbone, les laques de baryum, strontium, calcium, aluminium et leurs mélanges.

Comme silicates lamellaires, on peut citer les argiles, les talcs, les micas, les nacres, et leurs mélanges.

Les argiles sont des silicates mixtes d'origine naturelle ou synthétique renfermant plusieurs (deux ou plus) types de cations choisis parmi les métaux alcalins (par exemple Na, Li, K) ou alcalino-terreux (par exemple Be, Mg, Ca), les métaux de transition et l'aluminium.

Comme argiles utilisables dans l'invention, on peut citer par exemple le silicate de sodium et de magnésium (nom CTFA: Sodium magnesium silicate), les argiles de la famille du kaolin, telles que le kaolin ou la kaolinite, la dickite, la nacrite ; les argiles de la famille de l'halloysite, de la dombassite, de l'antigorite, de la benthiérine, de la pyrophyllite; les montmorillonites; la beidellite; les vermiculites; la stévensite; les hectorites ; les saponites ; les chlorites ; la sépiolite ; la smectite, ainsi que ces argiles modifiées chimiquement par exemple par les acides acryliques, les polysaccharides (par exemple la carboxyméthylcellulose) ou les cations organiques, et leurs mélanges.

Les talcs sont des silicates de magnésium hydratés comprenant le plus souvent du silicate d'aluminium. La structure cristalline du talc consiste en des couches répétées d'un sandwich de brucite entre des couches de silice.

Les micas sont des silicates d'aluminium comprenant éventuellement du fer et/ou des métaux alcalins. Ils ont la propriété de pouvoir se diviser en couches minces (environ 1 µm). Ils ont généralement une dimension allant de 5 à 150 µm, de préférence de 10 à 100 µm et mieux de 10 à 60 µm pour la plus grande dimension (longueur), et une hauteur (épaisseur) de 0,1 à 0,5 µm. On peut citer comme micas, la phlogopite, la muscovite, la fluorophlogopite, la vermiculite, et leurs mélanges. On peut citer aussi des argiles micacés tels que l'illite.

Par nacres, il faut comprendre des particules irisées, notamment produites par certains mollusques dans leur coquille ou bien synthétisées, servant à modifier la texture de la composition ainsi que l'effet de matité/brillance. Les nacres sont généralement des micas traités en surface pour obtenir cet effet irisé. Parmi les nacres utilisables dans l'invention, on peut citer par exemple les micas recouverts d'oxyde de titane, d'oxyde de fer, de pigment naturel et/ou d'oxychlorure de bismuth, tel que le mica-oxyde de titane (ou mica-titane) coloré ou non, et leurs mélanges.

Selon un mode particulièrement préféré de réalisation de la présente invention, les plaquettes sont choisies parmi le silicate de sodium et de magnésium ; le kaolin et la kaolinite ; les montmorillonites ; les hectorites; les talcs ; les micas ; les nacres, et leurs mélanges. De manière avantageuse, on utilise plus particulièrement dans la composition de l'invention, comme plaquette, le kaolin tel que le produit commercialisé sous la dénomination COSLIN C-100 par la société Engelhard ; le talc tel que ceux commercialisés sous les dénominations ROSE TALC et TALC SG-2000 par la société Nippon Talc; le mica tel que ceux commercialisés sous les dénominations MICA M RP et SILK MICA par la société Merck; les mica-titanes tels que le mica-oxyde de titane-oxyde de fer brun (CTFA: Mica / Iron oxides / Titanium dioxide) commercialisé sous la dénomination CLOISONNE ROUGE FLAMBE 440 X par la société Engelhard ; ou une hectorite modifiée, comme par exemple une bentone et plus particulièrement le mélange « cyclomethicone, Quaternium-18 hectorite, SD alcohol 40 » (85/10/5) (nom CTFA) commercialisé sous la dénomination Bentone Gel VS-5 par la société Rheox.

La quantité de plaquettes peut aller par exemple de 0,1 à 30 % en poids, de préférence de 0,25 à 25 % en poids et mieux de 0,5 à 10 % en poids par rapport au poids total de la composition.

### Phase huileuse

La phase huileuse de la composition selon l'invention représente généralement de 10 à 50 % en poids et de préférence de 15 à 30 % en poids par rapport au poids total de la composition.

La phase huileuse contient habituellement au moins une huile. Comme huiles utilisables dans la composition de l'invention, on peut citer par exemple :
- les huiles hydrocarbonées d'origine animale, telles que le perhydrosqualène;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de Parléam® ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les alcools gras alcoylés et notamment éthoxylés tels que l'oleth-12 ;
- les huiles fluorées partiellement hydrocarbonées et/ou siliconées comme celles décrites dans le document JP-A-2-295912. Comme huiles fluorées, on peut citer aussi le perfluorométhylcyclopentane et le perfluoro-1,3 diméthylcyclohexane, vendus sous les dénominations de "FLUTEC PC1® " et "FLUTEC PC3® " par la Société BNFL Fluorochemicals; le perfluoro-1,2-diméthylcyclobutane; les perfluoroalcanes tels que le dodécafluoropentane et le tétradécafluorohexane, vendus sous les dénominations de "PF 5050® " et "PF 5060® " par la Société 3M, ou encore le bromoperfluorooctyle vendu sous la dénomination "FORALKYL® " par la Société Atochem; le nonafluorométhoxybutane vendu sous la dénomination "MSX 4518® " par la Société 3M et le nonafluoroéthoxyisobutane; les dérivés de perfluoromorpholine, tels que la 4-trifluorométhyl perfluoromorpholine vendue sous la dénomination "PF 5052® " par la Société 3M;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les gommes telles que les gommes de silicone (diméthiconol) ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

### Phase aqueuse

La phase aqueuse de la composition de l'invention constitue en général de 30 à 85 % et de préférence de 60 à 75 % en poids par rapport au poids total de la composition.

### Additifs

La composition selon l'invention peut constituer notamment une émulsion huile-dans-eau. Elle contient alors de préférence au moins un émulsionnant choisi parmi ceux classiquement utilisé pour la préparation des émulsions H/E.

Comme émulsionnant, on peut citer par exemple les tensioactifs non ioniques, et notamment les esters de polyols et d'acide gras à chaîne saturée ou insaturée comportant par exemple de 8 à 24 atomes de carbone et mieux de 12 à 22 atomes de carbone, et leurs dérivés oxyalkylénés, c'est-à-dire comportant des unités oxyéthylénés et/ou oxypropylénés, tels les esters de glycéryle et d'acide gras en C₈-C₂₄, et leurs dérivés oxyalkylénés ; les esters de polyéthylène glycol et d'acide gras en C₈-C₂₄, et leurs dérivés oxyalkylénés; les esters de sorbitol et d'acide gras en C₈-C₂₄, et leurs dérivés oxyalkylénés; les esters de sucre (sucrose, glucose, alkylglucose) et d'acide gras en C₈-C₂₄, et leurs dérivés oxyalkylénés ; et leurs mélanges.

Comme ester de glycéryle et d'acide gras, on peut citer notamment le stéarate de glycéryle (mono-, di- et/ou tri-stéarate de glycéryle) ou le ricinoléate de glycéryle, et leurs mélanges.

Comme ester de polyéthylène glycol et d'acide gras, on peut citer notamment le stéarate de polyéthylène glycol (mono-, di- et/ou tri-stéarate de polyéthylène glycol), et plus spécialement le monostéarate de polyéthylène glycol 50 OE (nom CTFA: PEG-50 stearate), et leurs mélanges.

Comme ester d'acide gras et de glucose ou d'alkylglucose, on peut citer en particulier le palmitate de glucose, les sesquistéarates d'alkylglucose comme le sesquistéarate de méthylglucose, les palmitates d'alkylglucose comme le palmitate de méthylglucose ou d'éthylglucose, les esters gras de méthylglucoside et plus spécialement le diester de méthylglucoside et d'acide oléique (nom CTFA : Methyl glucose dioleate) ; l'ester mixte de méthylglucoside et du mélange acide oléique / acide hydroxystéarique (nom CTFA : Methyl glucose dioleate/hydroxystearate); l'ester de méthylglucoside et d'acide isostéarique (nom CTFA : Methyl glucose isostearate) ; l'ester de méthylglucoside et d'acide laurique (nom CTFA : Methyl glucose laurate) ; le mélange de monoester et de diester de méthylglucoside et d'acide isostéarique (nom CTFA : Methyl glucose sesqui-isostearate) ; le mélange de monoester et de diester de méthylglucoside et d'acide stéarique (nom CTFA : Methyl glucose sesquistearate) et en particulier le produit commercialisé sous la dénomination Glucate SS par la société AMERCHOL, et leurs mélanges.

Comme éthers oxyéthylénés d'acide gras et de glucose ou d'alkylglucose, on peut citer par exemple les éthers oxyéthylénés d'acide gras et de méthylglucose, et en particulier l'éther de polyéthylène glycol de diester de méthyl glucose et d'acide stéarique à environ 20 moles d'oxyde d'éthylène (nom CTFA : PEG-20 methyl glucose distearate) tel que le produit commercialisé sous la dénomination Glucam E-20 distearate par la société AMERCHOL ; l'éther de polyéthylène glycol du mélange de monoester et de diester de méthyl glucose et d'acide stéarique à environ 20 moles d'oxyde d'éthylène (nom CTFA : PEG-20 methyl glucose sesquistearate) et en particulier le produit commercialisé sous la dénomination Glucamate SSE-20 par la société AMERCHOL et celui commercialisé sous la dénomination Grillocose PSE-20 par la société GOLDSCHMIDT, et leurs mélanges.

Comme ester de sucrose, on peut citer par exemple le palmito-stéarate de saccharose, le stéarate de saccharose et le mono laurate de saccharose.

Selon leur nature, ces émulsionnants sont introduits dans la phase aqueuse ou dans la phase huileuse. Il peut y avoir également un émulsionnant dans la phase aqueuse et un autre émulsionnant dans la phase huileuse.

Selon un mode particulier de réalisation de l'invention, le ou les émulsionnants sont choisis parmi les tensioactifs non ioniques, et notamment les esters de polyol et d'acide gras et leurs dérivés oxyéthylénés, et plus particulièrement les esters de glucose ou d'alkylglucose et d'acide gras et leurs dérivés oxyéthylénés, et leurs mélanges.

La quantité d'émulsionnant(s) peut aller par exemple de 0,1 à 15 % en poids, de préférence 0,5 à 10 % en poids et mieux de 1 à 8 % en poids par rapport au poids total de la composition.

De façon connue, les compositions de l'invention peuvent contenir des adjuvants habituels dans les domaines considérés, tels que des actifs hydrophiles ou lipophiles, des conservateurs, des gélifiants, des antioxydants, des parfums, des solvants, des filtres, des colorants solubles, des agents basiques ou acides et encore des vésicules lipidiques. Ces adjuvants sont utilisés dans les proportions habituelles dans le domaine cosmétique, et par exemple de 0,01 à 30 % du poids total de l'émulsion, et ils sont, selon leur nature, introduits dans la phase aqueuse ou dans la phase huileuse de l'émulsion, ou encore dans des vésicules. Ces adjuvants ainsi que leurs concentrations doivent être tels qu'ils ne modifient pas la propriété recherchée pour l'émulsion de l'invention.

Comme actifs, on peut citer par exemple les hydratants tels que les polyols comme la glycérine et le sorbitol ; les agents kératolytiques ; les dépigmentants ; les amincissants, et tout actif approprié pour le but final de la composition.

Selon la fluidité de la composition que l'on souhaite obtenir, on peut y ajouter un ou plusieurs gélifiants hydrophiles ou lipophiles. Comme gélifiants hydrophiles, on peut citer par exemple les polymères carboxyvinyliques, tels que les carbomers ; les polyacrylamides et les polymères et copolymères d'acide 2-acrylamido 2-méthylpropane sulfonique), éventuellement réticulés et/ou neutralisés, comme le poly(acide 2-acrylamido 2-méthylpropane sulfonique) commercialisé par la société Hoechst sous la dénomination commerciale « Hostacerin AMPS » (nom CTFA: ammonium polyacryldimethyltauramide).

Comme gélifiants lipophiles, on peut citer les argiles modifiées telles que les bentones telles que le mélange « cyclomethicone, Quaternium-18 hectorite, SD alcohol 40 » (10/85/5) (nom CTFA) commercialisé sous la dénomination Bentone Gel VS-5 par la société Rheox; les organopolysiloxanes élastomères réticulés tels que ceux commercialisés sous les noms KSG6 de Shin-Etsu, Trefil E-505C ou Trefil E-506C de Dow-Corning, Gransil de Grant Industries (SR-CYC, SR DMF10, SR-DC556), ou ceux commercialisés sous forme de gels: KSG15, KSG17, KSG16, KSG18 de Shin-Etsu, Gransil SR SCYC gel, Gransil SR DMF 10 gel, Gransil SR DC 556 gel, SF 1204 et JK 113 de General Electric.

Ces gélifiants, lorsqu'ils sont présents, sont généralement utilisés à des concentrations allant de 0,1 à 7 % et de préférence de 0,1 à 5 % en poids de matière active par rapport au poids total de la composition.

Les compositions, objets de l'invention, trouvent leur application dans un grand nombre de traitements notamment cosmétiques ou dermatologiques, et elles peuvent ainsi constituer une composition cosmétique, notamment pour le traitement, la protection, le soin, le démaquillage et/ou le nettoyage de la peau, des lèvres et/ou des cheveux, et/ou pour le maquillage de la peau des lèvres, des cils et/ou du corps.

Les compositions selon l'invention peuvent par exemple être utilisées comme produits de soin, de démaquillage et/ou de nettoyage pour le visage sous forme de crèmes ou de laits ou comme produits de maquillage (peau, cils et lèvres) par incorporation de pigments ou de colorants, par exemple comme fonds de teint.

Aussi, l'invention a pour objet l'utilisation cosmétique de la composition telle que définie ci-dessus pour le traitement, la protection, le soin, le démaquillage et/ou le nettoyage de la peau, des lèvres et/ou des cheveux, et/ou pour le maquillage de la peau, des lèvres, des cils et/ou du corps.

L'invention a aussi pour objet un procédé de traitement cosmétique de la peau, y compris du cuir chevelu, des cheveux, des cils et/ou des lèvres, caractérisé par le fait qu'on applique sur la peau, les cheveux, les cils et/ou les lèvres, une composition telle que définie ci-dessus.

Les exemples qui suivent permettront de mieux comprendre l'invention, sans toutefois présenter un caractère limitatif. Les quantités indiquées sont en % en poids, sauf mention contraire.

L'exemple selon l'invention a été comparé dans un test sur un panel de 12 utilisateurs avec une composition identique mais ne contenant pas soit les plaquettes (exemple comparatif 1), soit les particules sphériques (exemple comparatif 2). L'application a été faite en comparatif en demi-visage, la composition de l'invention étant appliquée sur une moitié de visage tandis qu'un des exemples comparatifs était appliqué sur l'autre moitié du visage.

| Composition | Exemple 1 selon l'invention | Exemple comparatif 1 | Exemple comparatif 2 |
|---|---|---|---|
| *Phase A*1 | | | |
| Methyl-glucose sesquistearate (Glucate SS) | 2 % | 2 % | 2 % |
| Stearyl alcohol / Ceteareth-20 | 2 % | 2 % | 2 % |
| Conservateurs | 0,1 % | 0,1 % | 0,1 % |
| Octyl-2-dodecanol | 4% | 4% | 4% |

| *Phase A2* | | | |
|---|---|---|---|
| Cyclopentasiloxane | 5 % | 5 % | 5 % |

| *Phase B1* | | | |
|---|---|---|---|
| Glycérine | 3 % | 3 % | 3 % |
| Sodium magnesium silicate (argile) (plaquettes) | 0,5 % | - | 0,5 % |
| Eau déminéralisée | Qsp 100 % | Qsp 100 % | Qsp 100 % |
| Conservateurs | 0,25 % | 0,25 % | 0,25 % |

| *Phase B2* | | | |
|---|---|---|---|
| PEG-20 methyl-glucose sesquistearate (Glucamate SSE 20) | 3 % | 3 % | 3 % |
| Eau déminéralisée | 19 % | 19 % | 19 % |

| *Phase C* | | | |
|---|---|---|---|
| NYLON-12 (ORGASOL) (particules sphériques) | 2 % | 2 % | - |
| Fibres de polyamide (Nylon-66) (Polyamide 0,9 Dtex, 0,3 mm -Société Paul Bonte) | 10 % | 10 % | 10 % |

| *Phase D* | | | |
|---|---|---|---|
| Ammonium Polyacryloyldimethyltaurate (Hostacerin AMPS de Clariant) | 0,2 % | 0,2 % | 0,2 % |
| Qualité de la composition obtenue | - crémeuse, - fine et souple | fluide, glisse sur les doigts | - crémeuse et fine. |
| | | - boulo- | Par rapport |
| | - Application facile | chage de la composition - à | à l'ex. 1 : fibres plus persistantes |
| | - dépôt homogène | l'application | sur la peau, - application plus difficile, |
| | | | - dépôt moins homogène. |

Mode opératoire : Les phases A1 et A2 sont chauffées séparément (environ 70°C) sous agitation puis mélangées. De même, les phases B1 et B2 sont chauffées séparément (environ 70°C) sous agitation puis mélangées. Le mélange de B1 et B2 est ensuite versé dans le mélange de A1 et A2 sous agitation. On ajoute alors la phase D et on homogénéise.

Les trois compositions ont une texture fibreuse blanche. Le test met en évidence l'avantage de la composition selon l'invention, qui ne bouloche pas lors de l'application sur la peau et donne un dépôt plus homogène et plus facile que les compositions des exemples comparatifs.

## Revendications

1. Composition comprenant dans un milieu physiologiquement acceptable, une phase huileuse dispersée dans une phase aqueuse, des fibres, des particules sphériques et des plaquettes.

2. Composition selon la revendication 1, **caractérisée en ce que** les fibres ont une longueur (L) allant de 1 µm à 10 mm.

3. Composition selon la revendication 1 ou 2, **caractérisée en ce que** les fibres ont une section comprise dans un cercle de diamètre (D) allant de 1 nm à 100 µm.

4. Composition selon l'une quelconque des revendications précédentes, **caractérisée en ce que** les fibres ont un facteur de forme (L/D) allant de 5 à 150.

5. Composition selon l'une quelconque des revendications précédentes, **caractérisée en ce que** les fibres ont un titre allant de 0,15 à 30 deniers.

6. Composition selon l'une quelconque des revendications précédentes, **caractérisée en ce que** les fibres sont choisies parmi les fibres de soie, de coton, de laine, de lin, de cellulose extraites notamment du bois, des légumes ou des algues, de polyamide (Nylon® ), de cellulose modifiée, de poly-p-phénylène téréphtalamide, en acrylique, de polyoléfine, de verre, de silice, d'aramide, de carbone, de Téflon® , de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères, les fibres synthétiques résorbables, et leurs mélanges.

7. Composition selon l'une quelconque des revendications précédentes, **caractérisée en ce que** les fibres sont enrobées et/ou fonctionnalisées.

8. Composition selon l'une quelconque des revendications précédentes, **caractérisée en ce que** les fibres sont choisies parmi les fibres de polyamide, les fibres de poly-p-phénylène téréphtalamide, les fibres de coton et leurs mélanges.

9. Composition selon l'une quelconque des revendications précédentes, **caractérisée en ce que** les fibres sont présentes en une quantité allant de 0,1 à 50 % en poids et de préférence de 1 à 20 % en poids par rapport au poids total de la composition.

10. Composition selon l'une quelconque des revendications précédentes, **caractérisée en ce que** les particules sphériques sont des microsphères organiques ou minérales.

11. Composition selon l'une quelconque des revendications précédentes, **caractérisée en ce que** les particules sphériques ont une granulométrie allant de 0,1 µm à 250 µm.

12. Composition selon l'une quelconque des revendications précédentes, **caractérisée en ce que** les particules sphériques sont choisies parmi la poudre de silice ; les particules de polyamide ; les poudres de polyéthylène ; les microsphères à base de copolymères acryliques ; les poudres expansées ; les poudres de matériaux organiques naturels ; les microbilles de résine de silicone ; et leurs mélanges.

13. Composition selon l'une quelconque des revendications précédentes, **caractérisée en ce que** les particules sphériques sont présentes en une quantité allant de 0,1 à 30 % en poids et de préférence de 0,5 à 25 % en poids par rapport au poids total de la composition.

14. Composition selon l'une quelconque des revendications précédentes, **caractérisée en ce que** les plaquettes ont une longueur allant de 0,01 à 100 µm, une largeur de 0,01 à 100 µm et une hauteur allant de 0,1 à 1000 nm.

15. Composition selon l'une quelconque des revendications précédentes, **caractérisée en ce que** les plaquettes sont choisies parmi les pigments minéraux ou organiques, les silicates lamellaires, et leurs mélanges.

16. Composition selon l'une quelconque des revendications précédentes, **caractérisée en ce que** les plaquettes sont choisies parmi les oxydes de titane, de zirconium ou de cérium, les oxydes de zinc, de fer ou de chrome, le bleu ferrique, le noir de carbone, les laques de baryum, strontium, calcium, aluminium, et leurs mélanges.

17. Composition selon l'une quelconque des revendications précédentes, **caractérisée en ce que** les plaquettes sont choisies parmi les argiles, les talcs, les micas, les nacres et leurs mélanges.

18. Composition selon l'une quelconque des revendications précédentes, **caractérisée en ce que** les plaquettes sont choisies parmi le silicate de sodium et de magnésium ; le kaolin, les hectorites, les talcs, les micas, les micas recouverts d'oxyde de titane, d'oxyde de fer, de pigment naturel et/ou d'oxychlorure de bismuth, et leurs mélanges.

19. Composition selon l'une quelconque des revendications précédentes, **caractérisée en ce que** les plaquettes sont présentes en une quantité allant de 0,1 à 30 % en poids et de préférence de 0,25 à 25 % en poids par rapport au poids total de la composition.

20. Composition selon l'une quelconque des revendications précédentes, **caractérisée en ce que** la phase huileuse représente de 10 à 50 % en poids et de préférence de 15 à 30 % en poids par rapport au poids total de la composition.

21. Composition selon l'une quelconque des revendications précédentes, **caractérisée en ce qu'**elle constitue une émulsion Huile-dans-eau.

22. Composition selon la revendication précédente, **caractérisée en ce qu'**elle comprend au moins un émulsionnant choisi parmi les tensioactifs non ioniques.

23. Composition selon la revendication précédente, **caractérisée en ce que** le tensioactif non ionique est choisi parmi les esters de polyols et d'acide gras à chaîne saturée ou insaturée comportant par exemple de 8 à 24 atomes de carbone et mieux de 12 à 22 atomes de carbone, et leurs dérivés oxyalkylénés.

24. Composition selon la revendication précédente, **caractérisée en ce que** le tensioactif non ionique est choisi parmi les esters d'acide gras et de glucose ou d'alkylglucose, et leurs dérivés oxyéthylénés, et leurs mélanges.

25. Composition selon l'une quelconque des revendications 22 à 24, **caractérisée en ce que** la quantité d'émulsionnant(s) va de 0,1 à 15 % en poids et de préférence 0,5 à 10 % en poids par rapport au poids total de la composition.

26. Composition selon l'une quelconque des revendications précédentes, **caractérisée en ce qu'**elle constitue une composition cosmétique.

27. Utilisation cosmétique de la composition selon l'une quelconque des revendications 1 à 26, pour le traitement, la protection, le soin, le démaquillage et/ou le nettoyage de la peau, des lèvres et/ou des cheveux, et/ou pour le maquillage de la peau, des lèvres, des cils et/ou du corps.

28. Procédé de traitement cosmétique de la peau, des cheveux, des cils et/ou des lèvres, **caractérisé par le fait qu'**on applique sur la peau, les cheveux, les cils et/ou les lèvres, une composition selon l'une quelconque des revendications 1 à 26.

29. Utilisation cosmétique de l'association de plaquettes et de particules sphériques dans une composition cosmétique contenant des fibres, pour éviter que les fibres ne s'agglomèrent, pour éviter le boulochage de la composition et pour faciliter l'application de la composition sur les matières kératiniques et notamment la peau.

## Claims

1. Composition comprising, in a physiologically acceptable medium, an oily phase dispersed in an aqueous phase, fibres, spherical particles and platelets.

2. Composition according to Claim 1, **characterized in that** the fibres have a length (L) ranging from 1 µm to 10 mm.

3. Composition according to Claim 1 or 2, **characterized in that** the fibres have a cross section included within a circle of diameter (D) ranging from 1 nm to 100 µm.

4. Composition according to any one of the preceding claims, **characterized in that** the fibres have a shape factor (L/D) ranging from 5 to 150.

5. Composition according to any one of the preceding claims, **characterized in that** the fibres have a yarn count ranging from 0.15 to 30 denier.

6. Composition according to any one of the preceding claims, **characterized in that** the fibres are chosen from silk, cotton, wool or flax fibres, cellulose fibres extracted in particular from wood, plants or algae, polyamide (Nylon^{□}) fibres, modified cellulose fibres, poly-p-phenyleneterephthalamide fibres, acrylic fibres, polyolefin fibres, glass, silica or aramid fibres, carbon fibres, Teflon^{□} fibres, insoluble collagen fibres, polyester, polyvinyl chloride, polyvinylidene chloride, polyvinyl alcohol, polyacrylonitrile, chitosan, polyurethane or polyethylene phthalate fibres, fibres formed from a mixture of polymers, resorbable synthetic fibres, and mixtures thereof.

7. Composition acording to any one of the preceding claims, **characterized in that** the fibres are coated and/or functionalized.

8. Composition according to any one of the preceding claims, **characterized in that** the fibres are chosen from polyamide fibres, poly-p-phenyleneterephthalamide fibres and cotton fibres, and mixtures thereof.

9. Composition according to any one of the preceding claims, **characterized in that** the fibres are present in an amount ranging from 0.1% to 50% by weight and preferably from 1% to 20% by weight relative to the total weight of the composition.

10. Composition according to any one of the preceding claims, **characterized in that** the spherical particles are organic or mineral microspheres.

11. Composition according to any one of the preceding claims, **characterized in that** the spherical particles have a particle size ranging from 0.1 µm to 250 µm.

12. Composition according to any one of the preceding claims, **characterized in that** the spherical particles are chosen from silica powder; polyamide particles; polyethylene powders; microspheres based on acrylic copolymers; expanded powders; powders of natural organic materials; silicone resin microbeads; and mixtures thereof.

13. Composition according to any one of the preceding claims, **characterized in that** the spherical particles are present in an amount ranging from 0.1% to 30% by weight and preferably from 0.5% to 25% by weight relative to the total weight of the composition.

14. Composition according to any one of the preceding claims, **characterized in that** the platelets have a length ranging from 0.01 to 100 µm, a width of from 0.01 to 100 µm and a height ranging from 0.1 to 1 000 nm.

15. Composition according to any one of the preceding claims, **characterized in that** the platelets are chosen from mineral or organic pigments and lamellar silicates, and mixtures thereof.

16. Composition according to any one of the preceding claims, **characterized in that** the platelets are chosen from titanium oxide, zirconium oxide or cerium oxide, zinc oxide, iron oxide or chromium oxide, ferric blue, carbon black and barium, strontium, calcium and aluminium lakes, and mixtures thereof.

17. Composition according to any one of the preceding claims, **characterized in that** the platelets are chosen from clays, talcs, micas and nacres, and mixtures thereof.

18. Composition according to any one of the preceding claims, **characterized in that** the platelets are chosen from sodium magnesium silicate; kaolin, hectorites, talcs, micas, micas coated with titanium oxide, with iron oxide, with natural pigment and/or with bismuth oxychloride, and mixtures thereof.

19. Composition according to any one of the preceding claims, **characterized in that** the platelets are present in an amount ranging from 0.1% to 30% by weight and preferably from 0.25% to 25% by weight relative to the total weight of the composition.

20. Composition according to any one of the preceding claims, **characterized in that** the oily phase represents from 10% to 50% by weight and preferably from 15% to 30% by weight relative to the total weight of the composition.

21. Composition according to any one of the preceding claims, **characterized in that** it constitutes an oil-in-water emulsion.

22. Composition according to the preceding claim, **characterized in that** it comprises at least one emulsifier chosen from nonionic surfactants.

23. Composition according to the preceding claim, **characterized in that** the nonionic surfactant is chosen from polyol esters of fatty acids containing a saturated or unsaturated chain containing, for example, from 8 to 24 carbon atoms and better still from 12 to 22 carbon atoms, and oxyalkylenated derivatives thereof.

24. Composition according to the preceding claim, **characterized in that** the nonionic surfactant is chosen from fatty acid esters of glucose or of alkylglucose, and oxyethylenated derivatives thereof, and mixtures thereof.

25. Composition according to any one of Claims 22 to 24, **characterized in that** the amount of emulsifier(s) ranges from 0.1% to 15% by weight and preferably from 0.5% to 10% by weight relative to the total weight of the composition.

26. Composition according to any one of the preceding claims, **characterized in that** it constitutes a cosmetic composition.

27. Cosmetic use of the composition according to any one of Claims 1 to 26, for treating, protecting, caring for, removing make-up from and/or cleansing the skin, the lips and/or the hair, and/or for making up the skin, the lips, the eyelashes and/or the body.

28. Cosmetic process for treating the skin, the hair, the eyelashes and/or the lips, **characterized in that** a composition according to any one of Claims 1 to 26 is applied to the skin, the hair, the eyelashes and/or the lips.

29. Cosmetic use of the combination of platelets and spherical particles in a cosmetic composition containing fibres, to prevent the fibres from aggregating, to prevent the composition from pilling and to facilitate the application of the composition to keratin materials and especially the skin.

## Patentansprüche

1. Zusammensetzung, die in einem physiologisch akzeptablen Medium eine in einer wässerigen Phase dispergierte Ölphase, Fasern, sphärische Partikel und Plättchen enthält.

2. Zusammensetzung nach Anspruch 1, **dadurch gekennzeichnet, dass** die Fasern eine Länge (L) von 1 µm bis 10 mm aufweisen.

3. Zusammensetzung nach Anspruch 1 oder 2, **dadurch gekennzeichnet, dass** die Fasern einen Querschnitt besitzen, der in einen Kreis mit einem Durchmesser (D) von 1 nm bis 100 µm einbeschrieben werden kann.

4. Zusammensetzung nach einem der vorhergehenden Ansprüche, **dadurch gekennzeichnet, dass** die Fasern einen Formfaktor (L/D) im Bereich von 5 bis 150 besitzen.

5. Zusammensetzung nach einem der vorhergehenden Ansprüche, **dadurch gekennzeichnet, dass** die Fasern einen Titer von 0,15 bis 30 Denier aufweisen.

6. Zusammensetzung nach einem der vorhergehenden Ansprüche, **dadurch gekennzeichnet, dass** die Fasern unter den Seidenfasern, Baumwollfasern, Wollfasern, Leinenfasern, Fasern aus Cellulose, die insbesondere aus Holz, Gemüse oder Algen gewonnen wurden, Polyamidfasern (Nylon® ), Fasern aus modifizierter Cellulose, Poly-*p*-phenylenterephthalamidfasern, Acrylfasern, Polyolefinfasern, Glasfasern, Siliciumdioxidfasern, Aramidfasern, Kohlenstofffasern, Teflon® -Fasern, Fasern aus unlöslichem Collagen, Polyesterfasern, Polyvinylchloridfasern, Polyvinylidenchloridfasern, Polyvinylalkoholfasern, Polyacrylnitrilfasern, Chitosanfasern, Polyurethanfasern, Polyethylenphthalatfasern, Fasern, die aus einem Gemisch von Polymeren gebildet sind, resorbierbaren synthetischen Fasern und deren Gemischen ausgewählt sind.

7. Zusammensetzung nach einem der vorhergehenden Ansprüche, **dadurch gekennzeichnet, dass** die Fasern umhüllt und/oder funktionalisiert sind.

8. Zusammensetzung nach einem der vorhergehenden Ansprüche, **dadurch gekennzeichnet, dass** die Fasern unter den Polyamidfasern, Fasern aus Poly-*p*-phenylenterephthalamid, Baumwollfasern und deren Gemischen ausgewählt sind.

9. Zusammensetzung nach einem der vorhergehenden Ansprüche, **dadurch gekennzeichnet, dass** die Fasern in einer Menge von 0,1 bis 50 Gew.-% und vorzugsweise 1 bis 20 Gew.-%, bezogen auf das Gesamtgewicht der Zusammensetzung, enthalten sind.

10. Zusammensetzung nach einem der vorhergehenden Ansprüche, **dadurch gekennzeichnet, dass** die sphärischen Partikel organische oder anorganische Mikrosphären sind.

11. Zusammensetzung nach einem der vorhergehenden Ansprüche, **dadurch gekennzeichnet, dass** die sphärischen Partikel eine Korngröße von 0,1 bis 250 µm aufweisen.

12. Zusammensetzung nach einem der vorhergehenden Ansprüche, **dadurch gekennzeichnet, dass** die sphärischen Partikel unter Siliciumdioxidpulver; Polyamidpartikeln; Polyethylenpulvern; Mikrosphären auf der Basis von Acrylcopolymeren; expandierten Pulvern; Pulvern von natürlichen organischen Stoffen; Siliconharzmikrokugeln; und deren Gemischen ausgewählt sind.

13. Zusammensetzung nach einem der vorhergehenden Ansprüche, **dadurch gekennzeichnet, dass** die sphärischen Partikel in einer Menge von 0,1 bis 30 Gew.-% und vorzugsweise 0,5 bis 25 Gew.-%, bezogen auf das Gesamtgewicht der Zusammensetzung, enthalten sind.

14. Zusammensetzung nach einem der vorhergehenden Ansprüche, **dadurch gekennzeichnet, dass** die Plättchen eine Länge von 0,01 bis 100 µm, eine Breite von 0,01 bis 100 µm und eine Höhe von 0,1 bis 1.000 nm besitzen.

15. Zusammensetzung nach einem der vorhergehenden Ansprüche, **dadurch gekennzeichnet, dass** die Plättchen unter den anorganischen oder organischen Pigmenten, lamellaren Silicaten und deren Gemischen ausgewählt sind.

16. Zusammensetzung nach einem der vorhergehenden Ansprüche, **dadurch gekennzeichnet, dass** die Plättchen unter den Oxiden von Titan, Zirconium oder Cer, Oxiden von Zink, Eisen oder Chrom, Eisenblau, Ruß, Lacken von Barium, Strontium, Calcium oder Aluminium und deren Gemischen ausgewählt sind.

17. Zusammensetzung nach einem der vorhergehenden Ansprüche, **dadurch gekennzeichnet, dass** die Plättchen unter den Tonen, Talken, Glimmern, Perlglanzpigmenten und deren Gemischen ausgewählt sind.

18. Zusammensetzung nach einem der vorhergehenden Ansprüche, **dadurch gekennzeichnet, dass** die Plättchen unter Natriummagnesiumsilicat; Kaolin, Hectoriten, Talken, Glimmern, mit Titanoxid, Eisenoxid, Naturpigment und/oder Bismuthoxidchlorid überzogenen Glimmerpigmenten und deren Gemischen ausgewählt sind.

19. Zusammensetzung nach einem der vorhergehenden Ansprüche, **dadurch gekennzeichnet, dass** die Plättchen in einer Menge von 0,1 bis 30 Gew.-% und vorzugsweise 0,25 bis 25 Gew.-%, bezogen auf das Gesamtgewicht der Zusammensetzung, enthalten sind.

20. Zusammensetzung nach einem der vorhergehenden Ansprüche, **dadurch gekennzeichnet, dass** die Ölphase 10 bis 50 Gew.-% und vorzugsweise 15 bis 30 Gew.-%, bezogen auf das Gesamtgewicht der Zusammensetzung, ausmacht.

21. Zusammensetzung nach einem der vorhergehenden Ansprüche, **dadurch gekennzeichnet, dass** sie eine Öl-in-Wasser-Emulsion ist.

22. Zusammensetzung nach dem vorhergehenden Anspruch, **dadurch gekennzeichnet, dass** sie mindestens einen Emulgator enthält, der unter den nichtionischen grenzflächenaktiven Stoffen ausgewählt ist.

23. Zusammensetzung nach dem vorhergehenden Anspruch, **dadurch gekennzeichnet, dass** der nichtionische grenzflächenaktive Stoff unter den Estern von Polyolen und einer Fettsäure, die eine gesättigte oder ungesättigte Kette mit beispielsweise 8 bis 24 Kohlenstoffatomen und besser 12 bis 22 Kohlenstoffatomen besitzt, und deren alkoxylierten Derivaten ausgewählt ist.

24. Zusammensetzung nach dem vorhergehenden Anspruch, **dadurch gekennzeichnet, dass** der nichtionische grenzflächenaktive Stoff unter den Glucosefettsäureestern oder Alkylglucosefettsäureestern und deren alkoxylierten Derivaten und deren Gemischen ausgewählt ist.

25. Zusammensetzung nach einem der Ansprüche 22 bis 24, **dadurch gekennzeichnet, dass** der Mengenanteil des Emulgators oder der Emulgatoren im Bereich von 0,1 bis 15 Gew.-% und vorzugsweise 0,5 bis 10 Gew.-%, bezogen auf das Gesamtgewicht der Zusammensetzung, liegt.

26. Zusammensetzung nach einem der vorhergehenden Ansprüche, **dadurch gekennzeichnet, dass** es sich um eine kosmetische Zusammensetzung handelt.

27. Kosmetische Verwendung der Zusammensetzung nach einem der Ansprüche 1 bis 26 für die Behandlung, den Schutz, die Pflege, das Abschminken und/ oder die Reinigung der Haut, der Lippen und/oder der Haare und/oder zum Schminken der Haut, der Lippen, der Wimpern und/oder des Körpers.

28. Verfahren zur kosmetischen Behandlung der Haut, der Haare, der Wimpern und/oder der Lippen, **dadurch gekennzeichnet, dass** auf die Haut, die Haare, die Wimpern und/oder die Lippen eine Zusammensetzung nach einem der Ansprüche 1 bis 26 aufgetragen wird.

29. Kosmetische Verwendung der Kombination aus Plättchen und sphärischen Fasern in einer kosmetischen Zusammensetzung, die Fasern enthält, um zu verhindern, dass die Fasern agglomerieren, um die Klumpenbildung der Zusammensetzung zu vermeiden und das Aufbringen der Zusammensetzung auf die Keratinsubstanz und insbesondere die Haut zu vereinfachen.
